# FASCICULE DE BREVET EUROPEEN

(11) **EP 0 948 314 B1**
(45) Date de publication et mention de la délivrance du brevet: **02.05.2001**
(21) Numéro de dépôt: 97952062.4
(22) Date de dépôt: 15.12.1997
(51) Int. Cl.: A61K 7/48

(54) **COMPOSITION COSMETIQUE SOUS LA FORME D'UN GEL AQUEUX COMPRENANT UN COLORANT POLYMERIQUE**
KOSMETISCHE ZUBEREITUNG IM FORM EINES WÄSSRIGEN GELS WELCHES EINEN POLYMERISCHEN FARBSTOFF ENTHÄLT
COSMETIC COMPOSITION IN THE FORM OF AN AQUEOUS GEL CONTAINING A POLYMERIC COLOURING AGENT

(30) Priorité: 16.12.1996 FR 9615453
(43) Date de publication de la demande: 13.10.1999
(73) Titulaire: L'OREAL, 75008 Paris (FR)
(72) Inventeur: LEMANN, Patricia, F-94000 Créteil (FR); BARA, Isabelle, F-75013 Paris (FR)
(74) Mandataire: Lhoste, Catherine
(86) Numéro de dépôt international: FR9702303
(87) Numéro de publication internationale: WO9826757

(56) Documents cités:
- DE-A- 1 906 841
- FR-A- 2 695 033
- US-A- 4 740 581
- US-A- 4 803 255
- US-A- 4 804 719
- US-A- 4 808 677
- US-A- 5 102 980
- US-A- 5 194 463
- DATABASE WPI Week 9616 Derwent Publications Ltd., London, GB; AN 96-157114 XP002040376 & JP 08 041 140 A (TOYOBO KK)
- DATABASE WPI Week 9435 Derwent Publications Ltd., London, GB; AN 94-282958 XP002040377 & JP 06 210 708 A (TOPPAN PRINTING CO LTD)
- DATABASE WPI Week 8937 Derwent Publications Ltd., London, GB; AN 89-266929 XP002040378 & JP 01 193 380 A (PENTEL KK)
- DATABASE WPI Week 8523 Derwent Publications Ltd., London, GB; AN 78-07550 XP002040379 & JP 52 148 632 A (POLA KASEI KOGYO KK)

## Description

L'invention a pour objet de nouvelles compositions de maquillage de la peau, des yeux, des ongles, des lèvres comprenant au moins un colorant polymérique, ainsi qu'un procédé de maquillage des yeux, de la peau, des ongles, des lèvres.

On utilise habituellement comme matière colorante dans les compositions cosmétiques, soit des pigments minéraux ou organiques ou des laques, qui sont généralement insolubles dans les milieux aqueux et organiques, soit des colorants solubles dans les milieux aqueux ou organiques.

Les pigments et laques utilisés dans le domaine du maquillage sont d'origine et de nature chimique très diverses. Leurs propriétés physicochimiques: granulométrie, surface spécifique, densité etc... sont donc très différents. Ces différences se traduisent par des variations de comportement : la facilité de mise en oeuvre, de dispersion, la stabilité à la lumière, à la température et les propriétés mécaniques dans le cas des poudres sont propres à chaque matière colorante. Ces différences de comportement obligent fréquemment le formulateur à réadapter la composition de la formule lorsque la teinte de cette formule est modifiée. Elles limitent également la réalisation de certaines formules sous forme de monochromes. En particulier, certaines teintes sont irréalisables du fait que les pigments ou les laques qui permettraient théoriquement de les obtenir sont incompatibles entre eux.

On utilise de préférence les oxydes minéraux pour la coloration des compositions cosmétiques, car les laques organiques présentent un dégorgement trop important ce qui a en particulier pour conséquence de tâcher les lentilles oculaires dans le cas des eye-liners ou des mascaras. Cependant, ces pigments minéraux sont de couleur terne et fade, il est donc nécessaire d'en introduire une grande quantité dans les formules de mascara et d'eye-liner pour obtenir un trait suffisamment saturé. Ce fort pourcentage de particules minérales affecte fortement la brillance du film de maquillage. Dans le cas des rouges à lèvres, le problème est identique, les compositions brillantes doivent être formulées avec des petites quantités de pigments. Dans le domaine du maquillage, on associe donc souvent couvrance et saturation de la couleur, à la matité du film.

Dans le domaine du maquillage, seules les laques organiques permettaient jusqu'à présent d'obtenir des couleurs vives et intenses (les oxydes minéraux ont une couleur très fade). Cependant, la plupart des laques organiques présentent une très mauvaise tenue à la lumière, qui se traduit par une atténuation très nette de leur couleur dans le temps. Ce phénomène est encore aggravé lorsque ces laques sont associées à des pigments photoréactifs comme le dioxyde de titane. Or ces pigments photo-réactifs sont très largement utilisés dans le maquillage pour la protection contre les rayonnement ultra-violets. Par conséquent, l'utilisation des laques organiques est assez limitée, ce qui a pour conséquence une limitation des teintes réalisables.

L'association de certains pigments, en particulier les oxydes de fer, avec des huiles et certains actifs, dont notamment les vitamines, a souvent pour conséquence l'oxydation de ces huiles et de ces actifs.

Dans les compositions sous forme de gel, on observe fréquemment un phénomène de déstabilisation ionique du système gélifiant par les pigments lorsqu'ils se présentent sous la forme d'oxydes minéraux ou de laques.

Dans les compositions de mascara on utilise jusqu'à 10 % de pigments d'oxydes minéraux pour obtenir une coloration visible et acceptable sur les cils. Cependant, une aussi forte concentration de pigments entraîne une forte diminution des performances rhéologiques du mascara, et produit donc un maquillage hétérogène sur les cils. Les laques organiques, qui permettent l'obtention de couleurs beaucoup plus vives à des pourcentages inférieurs sont peu utilisables pour cette application car comme on l'a déjà indiqué, elles dégorgent facilement et donc risquent de tâcher les lentilles.

En outre, l'utilisation de colorants solubles dans des compositions de maquillage a pour effet d'accentuer les rides ou stries de la peau, le colorant venant se fixer, par migration, préférentiellement dans ces rides ou stries. Cet effet est contraire à celui d'atténuation des défauts de la peau que l'on cherche à obtenir par le maquillage. Ces colorants solubles présentent souvent l'inconvénient de laisser après le démaquillage des taches sur la peau et les ongles, avec lesquels ils ont une grande affinité.

Enfin, si les pigments minéraux doivent être utilisés en grandes quantités pour obtenir une coloration satisfaisante, en revanche, les colorants solubles ont un pouvoir colorant très élevé, ce qui conduit à les utiliser en quantités infinitésimales. Par conséquent leur dosage dans une formule cosmétique dans des conditions reproductibles est une opération extrêmement délicate.

FR-A-2695033 divulgue une polymère colorant mécanique hydrosoluble, en général apte pour l'utilisation cosmétique.

Il subsistait donc le besoin de disposer de matière colorante nouvelle permettant de pallier les inconvénients de l'art antérieur.

Aussi c'est avec étonnement que la demanderesse a découvert que l'utilisation d'un colorant polymérique appartenant à une famille particulière, pouvait permettre de préparer des compositions cosmétiques nouvelles, qui ne présentent plus les inconvénients de l'art antérieur.
En particulier, ces compositions peuvent être préparées suivant des formules invariables, quelle que soit la teinte souhaitée, avec une gamme de teintes très large et très nuancée, avec une grande intensité de coloration associée à un effet brillant si cela est souhaité, avec une bonne tenue à la lumière, y compris lorsque la composition comprend, en outre, des pigments photoréactifs.

En particulier, ces compositions ne relarguent pas de colorant sur la peau, ce qui évite l'apparition de stries sur la peau maquillée ou de tâches après le démaquillage. Elles permettent ainsi une coloration non permanente de la peau, des ongles, des lèvres, ou des cils : après le démaquillage, la peau, les ongles, les lèvres ou les cils ne sont pas colorés. On obtient ainsi un démaquillage net. On dit que les compositions "ne griffent pas".
Ces compositions sont préparées à partir de quantités de colorant polymérique suffisamment faibles pour ne pas déstabiliser les formules, mais en quantités suffisamment importantes pour être facilement dosées de façon reproductible.

L'invention a pour objet de nouvelles compositions de maquillage des yeux, de la peau, des ongles, des lèvres sous la forme d'un gel aqueux, caractérisées en ce qu'elles comprennent au moins un colorant polymérique.

L'invention a aussi pour objet l'utilisation d'un colorant polymérique, dans une composition de maquillage des yeux, de la peau, des ongles, des lèvres sous la forme d'un gel aqueux, pour former un film coloré sur la peau, les cils, les paupières, les ongles, les lèvres.

L'invention a encore pour objet l'utilisation d'un colorant polymérique dans une composition de maquillage des yeux, de la peau, des ongles, des lèvres sous la forme d'un gel aqueux, qui ne colore pas les cils, les yeux, les ongles, les lèvres après le démaquillage.

L'invention a également pour objet un procédé de maquillage des yeux, de la peau, des ongles, des lèvres, caractérisé par le fait que l'on applique sur les cils, les paupières, la peau, les ongles, les lèvres une composition telle que définie précédemment.

De préférence ces compositions comprennent au moins un gélifiant hydrophile.

La viscosité des compositions selon l'invention est préférentiellement supérieure à 1 Pa.s, plus préférentiellement à 3 Pa.s et encore plus préférentiellement à 5 Pa.s.

Par colorant polymérique, on entend un copolymère à base d'au moins deux monomères distincts dont l'un au moins est un colorant organique monomérique.

De tels colorants polymériques sont connus de l'homme du métier. On peut, par exemple, se référer aux documents : US-5,032,670; US-4,999,418; US-5,106,942 ; US-5,030,708; US-5,102,980; US-5,043,376; US-5,104,913; US-5,281,659, US-5,194,463; US-4,804,719; WO92/07913.
II est exposé dans ces documents que ces colorants ont la propriété de ne pas migrer, de ne pas exsuder, de ne pas être extractibles ou sublimables. Ils sont également réputés être stables à la lumière et présenter un fort pouvoir colorant. Toutefois, les milieux dans lesquels leurs mise en oeuvre est envisagée dans ces documents sont des milieux thermoplastiques, essentiellement pour la fabrication d'emballages. Rien ne laisse prévoir les propriétés étonnantes de ces polymères une fois qu'ils sont incorporés dans des compositions cosmétiques.

Les colorants polymériques utilisables dans la présente invention peuvent être de toute nature : polyester, polyamide, polyuréthanne, polyacrylique, poly(méth)-acrylique, polycarbonate, leurs mélanges.
Les colorants polymériques utilisables dans la présente invention sont de préférence des polymères polyester ou polyuréthanne. Ces colorants polymériques polyesters ou polyuréthanne peuvent être de type cristallin, semi-cristallin ou amorphe.

Habituellement, les colorants polymériques ont une viscosité intrinsèque d'au moins 0,20 (mesurée selon la méthode décrite dans le brevet US 4804719).
Ils peuvent résulter de la polymérisation de plusieurs monomères dont:
a) lorsqu'il s'agit d'un polymère de la famille des polyesters :
   (i) au moins un résidu acide di-carboxylique ;
   (ii) au moins un résidu diol ; et
   (iii) au moins un monomère colorant.
b) lorsqu'il s'agit d'un polymère de la famille des polyuréthannes :
   (i) au moins un résidu di-isocyanate;
   (ii) au moins un résidu diol ; et
   (iii) au moins un monomère colorant.

Les résidus acide di-carboxylique peuvent être de type aliphatique, alicyclique ou aromatique, comme par exemple les acides téréphtalique, isophtalique, ou bien encore les acides 1,4-cyclohexane dicarboxylique, 1,3-cyclohexane di-carboxylique, succinique, glutarique, adipique, sébacique, 1, 2-dodécanedioïque, 2,6-naphtalène dicarboxylique, etc.

Dans une variante, les résidus acide dicarboxylique peuvent porter au moins un groupement sulfonique et être choisis parmi les diacides cycloaliphatiques comme le sulfo diacide 1,4-cyclohexane carboxylique, ou bien encore parmi les diacides aromatiques tels que les acides sulfophtaliques, l'acide 4-sulfonaphtalène-2,7-dicarboxylique, le sel de sodium de l'acide sulfo-5-phtalique.
Les résidus di-isocyanate peuvent être de type aliphatique, alicyclique ou aromatique, comme par exemple le 2,4-tolylène di-isocyanate, le 2,6-tolylène di-isocyanate, le 4,4'-biphénylène di-isocyanate, le p-xylène di-isocyanate, le méthylène di-p-phényl di-isocyanate, le p-phénylène di-isocyanate, le m-phénylène di-isocyanate, l'hexaméthylène di-isocyanate, l'isophorone di-isocyanate, etc.
Les résidus diols peuvent être choisi par exemple parmi l'éthylène glycol, le 1,2-propane diol, le 1,3-propane diol, le 2-méthyl-1,3-propanediol, le 1,4-butane diol, le 2,2-diméthyl-1,3-propanediol, le 1,6-hexanediol, le 1,10-décane diol, le 1,12-dodécane diol, le 1,2-cyclohexane diol, le 1,4-cyclohexanediol, le 1,2-cyclohexane diméthanol, le x,8-bis(hydroxyméthyl)-tricyclo-[5.2.1.0]décane, dans lequel x représente 3, 4 ou 5; les diols comprenant au moins un atome d'oxygène dans la chaîne comme par exemple le diéthylène glycol, le triéthylène glycol, le dipropylène glycol, le 1,3-bis (2-hydroxyéthyl) benzène, le 1,4-bis (2-hydroxyéthyl)benzène, etc.

En règle générale, ces diols comprennent 2 à 18 et préférentiellement 2 à 12 atomes de carbone.

Les monomères colorants organiques selon l'invention doivent comporter au moins deux substituants susceptibles de réagir avec au moins l'un des autres monomères employés pour la préparation du colorant polymérique et doivent être stables à la température et dans les conditions de préparation dudit polymère.

Hormis ces deux conditions préliminaires, la nature chimique des monomères colorants n'a pas d'importance pour la réalisation de l'invention. Ceux-ci peuvent par exemple, être choisis parmi les anthraquinones, les méthines, bis-méthines, les aza-méthines, les arylidènes, les 3H-dibenzo[7,i-j] isoquinolines, les acides 2,5-diarylaminotéréphtaliques et leurs esters, les phtaloylphénothiazines, les phtaloylphénoxazines, les phtaloylacridone, les anthrapyrimidines, les anthrapyrazoles, les phtalocyanines, les quinophtalones, les indophénols, perinones, les nitroarylamines, benzodifurane, les 2 H-1-benzopyran-2-one, les quinophtalones, les perylènes, les quinacridones, les triphénodioxazines, les fluoridines, les 4-amino-1,8-naphtalimides, les thioxanthrones, les benzanthrones, les indanthrones, les indigo, thioindigo, xanthène, acridine, azine, oxazine, etc.
On pourra se référer aux brevets US-4,267,306; US-4,359,570; US-4,403,092 ; US-4,617,373; US-4,080,355; US-4,740,581; US-4,116,923 ; US-4,745,173 ; US-4,804,719, US-5,194,463 ; WO92/07913 pour trouver des exemples de monomères colorants utilisables dans la préparation des colorants polymériques.

Les substituants portés par le monomère colorant et susceptibles de réagir avec les autres monomères peuvent par exemple être choisis parmi les groupements suivants:
- hydroxy,
- carboxy, ester, amino, alkylamino : 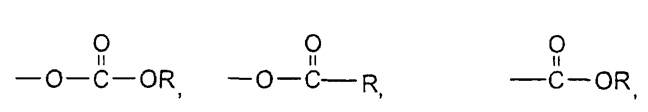 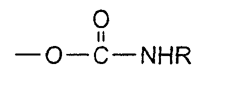
dans lesquels R représente un groupement choisi parmi les alkyles, les aryles. De préférence R est choisi parmi les groupements alkyles en C1-C8 et le phényle. Encore plus préférentiellement R est choisi parmi les groupements méthyle, éthyle et phényle.

Habituellement, le colorant polymérique comprend au moins 5 % en poids de monomère colorant, et n'en comprend pas plus de 55% en poids.
De préférence, le pourcentage en poids de monomère colorant par rapport au poids total du copolymère va de 10 à 40 %.

Pour la préparation des colorants polymériques, on peut se reporter aux procédés décrits dans les documents: US-5,032,670; US-4,999,418; US-5,106,942; US-5,030,708; US-5,102,980; US-5,043,376, US-5,194,463; US-4,804,719; WO92/07913.

Selon un mode particulier de l'invention, on peut utiliser un polymère colorant sulfopolyester dispersible dans l'eau ayant des groupes de liaison comprenant au moins environ 20 % en moles de carbonyloxy et jusqu'à environ 80 % en moles de carbonylamido, ledit polymère contenant des groupes sulfonate hydrosolubilisants et ayant d'environ 0,01 à environ 40 % en moles, sur la base du total de tous les équivalents d'hydroxy, de carboxy ou d'amino réactifs, de colorant comprenant un ou plusieurs composés organiques thermostables ayant initialement au moins un groupe condensable, que l'on a fait réagir sur ou dans le tronc polymère. Les équivalents susmentionnés englobent leurs divers dérivés condensables, y compris des carbalcoxy, carbaryloxy, N-alkylcarbamyloxy, acyloxy, chlorocarbonyle, carbamyloxy, N-(alkyl)₂ carbamyloxy, alkylamino, N-phénylcarbamyloxy, cyclohexanoyloxy et carbocyclohexyloxy.

Dans une forme préférée de la présente invention, le polymère colorant contient des groupes de liaison carbonyloxy dans la structure moléculaire linéaire, où jusqu'à 80 % desdits groupes de liaison peuvent être des groupes de liaison carbonylamido, le polymère ayant une viscosité inhérente d'environ 0,1 à environ 1, mesurée dans une solution à 60-40 parties en poids de phénol/tétrachloroéthane, à 25 °C et à une concentration de 0,25 gramme de polymère dans 100 ml du solvant, le polymère contenant des proportions essentiellement équimolaires d'équivalents d'acide (100 pour cent en moles) par rapport aux équivalents d'hydroxy et d'amino (100 pour cent en moles), le polymère comprenant les résidus de réaction des réactifs (a), (b), (c), (d) et (e) suivants ou de leurs dérivés générateurs d'ester ou générateurs d'estéramide:
(a) au moins un acide dicarboxylique difonctionnel;
(b) d'environ 4 à environ 25 pour cent en moles, sur la base d'un total de tous les équivalents d'acide, d'hydroxyle et d'amino étant égal à 200 pour cent en moles, d'au moins 1 sulfomonomère difonctionnel contenant au moins un groupe sulfonate cationique relié à un noyau aromatique ou cycloaliphatique où les groupes fonctionnels sont des hydroxy, carboxyle ou amino;
(c) au moins un réactif difonctionnel choisi parmi un glycol ou un mélange d'un glycol et d'une diamine ayant deux groupes -NRH, le glycol contenant deux groupes -CH₂-OH dont
   (1) au moins 10 pour cent en moles, sur la base du pourcentage total en moles d'équivalents d'hydroxy ou d'hydroxy et d'amino, sont un polyéthylèneglycol de formule structurale:

   H-(OCH₂-CH₂)ₙ-OH

   n étant un entier de 2 à environ 20 ou
(2) d'environ 0,1 à moins d'environ 15 pour cent en moles, sur la base du pourcentage total en moles d'équivalents d'hydroxy ou d'hydroxy et d'amino, sont un polyéthylèneglycol de formule structurale:

   H-(OCH₂-CH₂)ₙ-OH

   n étant un entier compris entre 2 et environ 500, et à condition que le pourcentage en moles dudit polyéthylèneglycol dans ladite gamme soit inversement proportionnel à la valeur de n dans ladite gamme ;
(d) de zéro à au moins un réactif difonctionnel choisi parmi un acide hydroxycarboxylique ayant un groupe -C(R)₂-OH, un acide aminocarboxylique ayant un groupe -NRH, et un amino-alcool ayant un groupe - C(R)₂-OH et un groupe -NRH, ou des mélanges desdits réactifs difonctionnels ; où chaque R dans les réactifs (c) ou (d) est un atome H ou un groupe alkyle ayant de 1 à 4 atomes de carbone ; et
(e) d'environ 0,1 % en moles à environ 15 % en moles, sur la base d'un total de tous les équivalents d'acide, d'hydroxyle et d'amino étant égal à 200 % en moles, de colorant ayant au moins un groupe acide, hydroxy ou amino que l'on a fait réagir sur ou dans la chaîne polymère.

Avantageusement, le polymère colorant sulfopolyester hydrodispersible comprend:
(a) un monomère acide comprenant de 75 mole % à 84 mole % d'acide isophtalique et de 25 mole % à 16 mole % de sel de sodium de l'acide sulfo-5-isophtalique,
(b) un monomère glycol comprenant de 45 à 60 mole % de diéthylène glycol et de 55 à 40 mole % de 1,4-cyclohexanediméthanol ou d'éthylèneglycol ou leur mélange,
(c) de 0,5 à 10 mole % de monomère colorant
   De tels polymères colorant sont décrits dans le brevet US 4804719.

Des colorants polymérique polyuréthane peuvent être par exemple les polymères décrits dans le brevet US-A-5194463 et répondant à la formule (I) suivante : 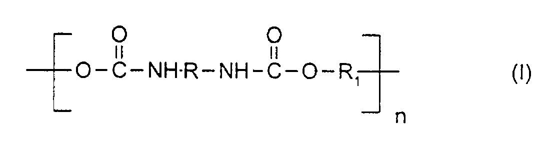 dans laquelle:
R est un radical divalent choisi parmi les radicaux alkylène en C2-C10, cycloalkylène en C3-C8, arylène, alkylène(C1-C4)-arylène-alkylène(C1-C4), alkylène(C1-C4)-cycloalkylène(C3-C8)-alkylène(C1-C4), alkylène(C1-C4)-1,2,3,4, 5,6,7-octahydronaphtalène-2,6-diyl-alkylène(C1-C4),
R¹ est un radical divalent organique comprenant (a) de 1 à 100 moles % de diol organique colorant dans lequel les groupes hydroxyle dudit diol sont liés par un motif alkylène au reste du composé colorant, et (b) de 0 à 99 moles % de diols organiques de formule HO-R²-OH, dans laquelle R² est un radical divalent choisi parmi les radicaux alkylène en C2-C18, cycloalkylène en C3-C8, alkylène(C1-C4)-arylène-alkylène(C1-C4), alkylène(C1-C4)-cycloalkylène(C3-C8)-alkylène (C1-C4), aikylène(C1-C4)-1,2,3,4,5,6,7-octahydronaphtalène-2,6-diyl-alkylène (C1-C4), alkylène(C2-C4)-O-alkylène(C2-C4), alkylène(C2-C4)-S-alkylène(C2-C4), alkylène(C2-C4)-O-alkylène(C2-C4)-O-alkylène (C2-C4),
   et n est égal ou supérieur à 2.
   De préférence, R1 comprend de 5 à 50 moles pourcent de diol colorant, et n va de 2 à 100.

Pour les polyuréthanes, les monomères diols colorants sont choisis parmi une variété de classes de chromophores. Ces classes de chrompophores peuvent être choisis parmi les anthraquinones, les méthines, bis-méthines, les azo-méthines, les arylidènes, les 3H-dibenzo[7,i-j] isoquinolines, les phtaloylphénoxazines, les phtaloylacridone, les anthrapyrimidines, les anthrapyrazoles, les phtalocyanines, les quinophtalones, les indophénols, perinones, les nitroarylamines, benzodifurane, les 2 H-1-benzopyran-2-one, les quinophtalones, les triphénodioxazines, les fluoridines, les benzanthrones, les indigo, thioindigo, xanthène, acridine, azine, oxazine, etc, Ces monomères colorants doivent porter au moins deux groupements hydroxyles.
On pourra se référer aux brevets US-5,194,463 pour trouver des exemples de monomères colorant.

Le colorant polymérique utilisable dans la présente invention peut se présenter sous forme brute, sous la forme d'une poudre dispersible d'une solution en milieu aqueux ou d'une dispersion en milieu aqueux.

Une dispersion en milieu aqueux d'un colorant polymérique insoluble dans l'eau peut être préparée de façon connue à partir d'eau, de colorant polymérique brut tel que décrit dans les documents US-5,032,670 ; US-4,999,418 ; US-5,106,942; US-5,030,708 ; US-5,102,980; US-5,043,376, US-5,194,463. Par exemple on peut partir du colorant polymérique sous forme de poudre ou de granulé, et d'au moins un tensio-actif ionique, de préférence un tensio-actif anionique ou amphotère. Les tensio-actifs anioniques et amphotères peuvent être choisis préférentiellement parmi les sels alcalins d'acides gras en C12-C24, les phosphatides de soja, les phospholipides, les lysophospholipides. Ces tensio-actifs ioniques sont introduits en quantités préférentiellement comprises entre 0,5 et 30%, et encore plus préférentiellement entre 1 et 10% en poids par rapport au poids du colorant polymérique. De façon préférentielle, ces dispersions comprennent en outre au moins un tensioactif non-ionique, que l'on choisit avantageusement parmi les dérivés polyoxyéthylénés ayant un poids moléculaire supérieur à 300, préférablement aux alentours de 1000 à 15000 et une balance hydrophile-lipophile (ou balance HLB) supérieure ou égale à 10.

Par exemple, on peut préparer une dispersion aqueuse de colorant polymérique en suivant les étapes suivantes:
(i) préparation d'une émulsion huile dans eau à partir d'eau, d'une solution du colorant polymérique dans un solvant organique volatile dans lequel il est soluble, en présence d'au moins un tensio-actif ionique et éventuellement d'un tensio-actif non-ionique ;
(ii) évaporation du solvant organique volatile.

Le solvant organique volatile utilisable pour la mise en oeuvre de ce procédé doit être non miscible à l'eau et susceptible de solubiliser le polymère. De préférence son point d'ébullition est inférieur à 100°C. Il peut par exemple être choisi parmi: les solvants hydrocarbonés comme le n-hexane, le cyclohexane, le cyclopentane ; les solvants chlorés comme le chlorure de méthylène, le chloroforme ; les alkyl esters d'acides carboxyliques, comme l'acétate d'éthyle ; les dialkyléthers comme le diisopropyléther. On utilise préférentiellement pour la mise en oeuvre de ce procédé un mélange de solvants comprenant, outre les solvants décrits ci-dessus un solvant volatile, polaire, miscible à l'eau, comme l'acétone ou un alcanol de faible poids moléculaire.

Pour plus d'information sur un tel procédé, on peut se référer aux brevet US 5,043,376 et US 5,104,913.

L'eau des dispersions aqueuses décrites ci-dessus peut ensuite être évaporée, par exemple par atomisation ou par lyophilisation, afin d'obtenir une poudre d'une composition de colorant polymérique dispersible dans tous les milieux.

En employant des méthodes connues de l'homme du métier, on prépare un gel comprenant au moins un colorant polymérique tel que décrit ci-dessus. Le colorant polymérique peut être incorporé sous forme de solution, de dispersion aqueuse ou tel quel, sous forme de poudre dispersible ou de poudre brute. Par exemple, on prépare une composition aqueuse à partir d'une dispersion aqueuse de colorant polymérique telle que décrite ci-dessus et de composants cosmétiques habituels dont au moins un agent gélifiant et on procède à la gélification. On peut également préparer une composition cosmétique aqueuse sous forme liquide comprenant au moins un agent gélifiant, dans laquelle on disperse ou on solubilise au moins un colorant polymérique sous forme de poudre tel que décrit ci-dessus et on procède à la gélification.

On peut utiliser dans la préparation des gels aqueux selon l'invention tous les gélifiants connus de l'homme du métier. On peut citer par exemple: les organopolysiloxanes; les polymères ou copolymères acryliques et polyméthacryliques, les polyacrylamides ; les dérivés de polysaccharides, tels que les dérivés cellulosiques comme la carboxy-méthylcellulose, l'hydroxyméthylcellulose, l'hydroxypropylcellulose, la cellulose microcristalline ; les polymères de xanthane, de gellane, de rhamsan, les alginates, la maltodextrine, l'amidon et ses dérivés, l'acide hyaluronique et ses sels, la gomme de karoya, la farine de caroube, les dérivés de guar notamment l'hydropropylguar ;les polyéthylèneglycols (PEG) et leurs dérivés, les polyvinylpyrrolidones et leurs dérivés.

Les organopolysiloxanes utilisables dans l'invention sont rendus solubles soit par modification chimique, soit de façon physique par adjonction d'un composé par exemple un tensioactif. Ils peuvent être utilisés seuls ou en mélange.

En particulier, comme organopolysiloxane solubilisé chimiquement, on peut utiliser un copolymère de siloxane et de protéine, hydrolysée ou non, comme décrit notamment dans le document EP-A-540 357. Des exemples de tels copolymères sont les copolymères de polysiloxane ou dérivés liés de manière covalente par greffage à une protéine hydrolysée ou non telle que la caséine, l'élastine, le collagène ou une protéine de blé ou de soja. On peut par exemple utiliser un siloxane phosphaté greffé sur une protéine de blé hydrolysée.

Ces copolymères greffés sont notamment vendus par la société CRODA sous la dénomination Crodasone W, par la société PHOENIX sous la dénomination Pecosil SWP 83 ou la dénomination Non-P-Silicon Protein.

Comme autre organopolysiloxane solubilisé chimiquement, utilisable dans le gel de l'invention, on peut également citer les diméthicones copolyols (nomenclature CFTA) et leurs dérivés.

Des diméthicones copolyols ont en particulier été présentés par la société DOW CORNING lors du 17ème congrès international de l'I.F.S.C.C. d'octobre 1992 et rapportés dans l'article "water-soluble dimethicone copolyol waxes for personal care industry", de Linda Madore et. al. p. 1 à 3.
Ces diméthicones copolyols sont des polydiméthylsiloxanes (PDMS) comportant une ou plusieurs fonctions ethers, solubles dans l'eau (oxyalkylène, notamment oxyéthylène et/ou oxypropylène).
De tels diméthicones copolyols sont notamment vendus par la société GOLDSCHMIDT sous la dénomination ABIL B8851 ou ABIL B88183.
On peut citer aussi les composés KF 351 à 354 et KF 615 A vendus par la société SHIN ETSU ou la DMC 6038 de la société WACKER.

Les dérivés de diméthicones copolyols utilisables dans l'invention sont en particulier les diméthicones copolyols à groupement phosphate, sulfate, chlorure de myristamide propyl diméthylammonium, stéarate, amine, glycomodifié, etc... .

On utilise comme dérivés de diméthicones copolyols notamment les composés vendus par la société SILTECH sous la dénomination Silphos A100, Siltech amine 65, Silwax WDIS, myristamido silicone quat, ou par la société PHOENIX sous la dénomination Pecosil PS 100.

On peut utiliser également les dérivés vendus par la société WACKER sous la dénomination VP 1661, ou par la société DOW CORNING sous la dénomination 2501 cosmetic wax.

Comme organopolysiloxane solubilisé physiquement, utilisable dans l'invention, on peut citer des organopolysiloxanes solubilisés à l'aide d'un tensioactif de valeur HLB supérieure à 8, de préférence supérieure ou égale à 10 ; comme cela est bien connu, on entend par HLB (Hydrophilic-Lipophilic Balance, en terminologie anglo-saxonne ou équilibre hydrophile-lipophile) l'équilibre entre la dimension et la force du groupe hydrophile et la dimension et la force du groupe lipophile de l'agent tensioactif. Ces organopolysiloxanes se présentent sous forme d'émulsion ou de microémulsion contenant de 10 % à 90 % en poids d'organopolysiloxane par rapport au poids total de l'émulsion ou microémulsion.

De préférence, ces organopolysiloxanes sont choisis dans le groupe comprenant les polydiméthylsiloxanes (PDMS), éventuellement de type gomme, pris seuls ou en mélange avec des cyclométhicones (CTFA).

Des exemples d'organopolysiloxane solubilisés physiquement utilisables dans l'invention sont notamment les SM2140 ou SM 2112 de DOW CORNING, la MFF 5015-70 de SILTECH, le TP 503 ou le TP 233 B de UNION CARBIDE.

Les tensioactifs de valeur de HLB (équilibre hydrophile/lipophile) supérieure à 8 sont notamment des éthers d'alcool gras en C₆ à C₂₄ et de polyoxyde d'éthylène. Ils sont incorporés en général à raison de 0,5 % à 30 % en poids par rapport au poids total de l'émulsion ou microémulsion. Ce sont par exemple les polyoxyéthyléné 12 cétyl/stéaryl éther ou polyoxyéthyléné 20 cétyl/stéaryl éther.

La viscosité du gel de l'invention dépend de la nature et de la quantité du ou des polymères hydrophiles, utilisés en tant que gélifiants. Ces gélifiants sont par exemple les polymères ou copolymères acryliques et polyméthacryliques comme les copolymères acide acrylique/acrylate d'éthyle et les polymères carboxyvinyliques. Des exemples de tels polymères ou copolymères sont notamment les "carbomer" (CTFA) vendus par la société GOODRICH sous la dénomination Carbopol ou le polyglycérylméthacrylate vendu par la société GUARDIAN sous la dénomination Lubragel ou encore le polyglycérylacrylate vendu sous la dénomination Hispagel par la société HISPANO CHIMICA ou enfin le mélange polyacrylamide/C₁₃-C₁₄ Isoparaffin/Laureth7 vendu par la société
SEPPIC sous la dénomination Sepigel.

De manière préférée, on utilise en tant que polymère hydrophile un dérivé acrylique, tel qu'un carbomer, vendu sous le nom de Carbopol par la Société GOODRICH (dans ce cas, et de manière classique, on neutralise le polymère à l'aide d'un agent basique comme, par exemple la triéthanolamine ou la soude), un polyglycéryl méthacrylate comme celui vendu sous la marque Lubragel par la Société GUARDIAN, ou des polysaccharides tels que des dérivés de cellulose.

Suivant la nature chimique du gélifiant, on utilise en général de 0,01 à 50% en poids de gélifiant par rapport au poids total de la composition. A titre indicatif, on peut donner les proportions suivantes:

Les teneurs en copolymères de siloxane et de protéine, hydrolysée ou non, sont de préférence choisies de 1 % à 50 % et, de manière encore plus préférée, de 10% à 50% en poids par rapport au poids total du gel.

On utilise les diméthicones copolyols et leurs dérivés dans la composition conforme à l'invention à des concentrations de préférence choisies de 0,5 % à 20 % en poids par rapport au poids total du gel.

On utilise ces organopolysiloxanes solubilisés physiquement à des teneurs choisies de préférence de 0,02 % à 20 % en poids par rapport au poids total du gel.

Les polymères hydrophiles sont généralement présents à raison de 0,01 % à 30 %, et de préférence de 0,1 % à 5 % en poids par rapport au poids total du gel.

Un gel selon l'invention peut être utilisé tel quel comme mascara, comme produit de maquillage des yeux (mascara, fard à paupières, eye-liner), des lèvres (soin des lèvres, rouge à lèvres) ou de la peau (fond de teint, fard à joue, des ongles (vernis à ongles) . Il peut également être incorporé comme phase aqueuse d'une émulsion. Par exemple on peut préparer une émulsion eau-dans-huile, huile-dans-eau, eau-dans-cire, cire-dans-eau, une émulsion triple, à partir d'huile et/ou de cire et d'au moins un gel selon l'invention.

Les colorants polymériques utilisables dans les gels aqueux selon l'invention peuvent être introduits en quantités allant de 0,1 à 20%.

Les colorants polymériques décrits ci-dessus ont l'avantage de présenter la même structure chimique générale et les mêmes propriétés physico-chimiques, dans des gammes de couleurs très différentes. Ainsi, un pigment rouge aura le même comportement qu'un pigment de couleur jaune. Cette homogénéité de comportement permet la fabrication des compositions de maquillages sous forme de monochromes. Lors du mélange des monochromes pour la réalisation d'une teinte, on n'observe aucun problème d'incompatibilité entre les différentes couleurs.

Ces colorants ont un pouvoir colorant supérieur aux oxydes minéraux et proche de celui des laques organiques : 4 à 5 % suffisent à donner un mascara de couleur vive et intense. Les problèmes de rhéologie sont donc fortement atténués. De plus, ces polymères colorés ne relarguent pas de colorant. Il n'y a donc aucun problème d'apparition de stries, de tâchage de la peau ou des lentilles oculaires.

Ces colorants polymériques, utilisés dans les gels selon l'invention ont un pouvoir colorant très proche de celui des laques organiques, et présentent une très grande stabilité à la lumière, ceci même en présence de dioxyde de titane ou de zinc. On peut donc les utiliser à la place des laques organiques dans toutes les formules de maquillage. Leur grande stabilité à la lumière permet de présenter les compositions cosmétiques les comprenant dans des emballages transparents. Ainsi, l'utilisatrice peut choisir avec plus de précision la couleur du produit qu'elle achète. En outre, ils ne déstabilisent pas les gels, même lorsqu'ils sont utilisés en quantité importante et ils n'interfèrent pas avec les huiles et/ou les actifs.

Ces colorants polymériques de couleurs vives et intenses, ont la particularité d'être suffisamment couvrants pour qu'une petite quantité soit suffisante pour colorer une formule cosmétique. La brillance du film est donc nettement améliorée. On obtient un maquillage à la fois couvrant, saturé et brillant.

Les émulsions comprenant un gel selon l'invention comprennent préférentiellement au moins un émulsionnant. Cet émulsionnant peut être un tensio-actif choisi parmi les tensio-actifs non-ioniques, cationiques, anioniques ou amphotères. Outre les tensio-actifs décrits ci-dessus pour la préparation des dispersions aqueuses de colorant polymérique, on peut citer comme exemple de tensioactifs utilisables dans l'invention : les émulsionnants à base de silicone et les émulsionnants lipidiques comme les alcools gras, les acides gras, les esters de glycérol, les esters de sorbitan, les esters de méthylglycoside et les esters de saccharose.

La phase grasse comprend au moins une huile éventuellement volatile et/ou une cire.

Selon l'invention, la cire peut être une cire animale, végétale, minérale ou synthétique. Parmi les cires animales, on peut notamment citer les cires d'abeilles, la cire de baleine. Parmi les cires végétales, on peut citer, entre autres, les cires de Carnauba, de Candellila, d'Ouricoury, les cires de fibres de liège, les cires de canne à sucre et les cires du Japon. Parmi les cires minérales, on peut citer, en particulier, les cires de paraffine, la lanoline, les cires microcristallines, les cires de lignite et les ozokérites. Parmi les cires synthétiques, on peut citer, notamment, les cires de polyéthylène et les cires obtenues par synthèse de Fisher et Tropsch. Toutes ces cires sont bien connues de l'Homme de l'Art.

On peut citer parmi les huiles utilisables dans la présente invention, l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide des huiles d'hydrocarbures telles que des huiles de paraffine, le squalane, la vaseline; des esters tels que le myristate d'isopropyle, le palmitate d'isopropyle, le

stéarate de butyle, le laurate d'hexyle, l'isononanoate d'isononyle, le palmitate de 2-éthyl hexyle, le laurate de 2-hexyl décyle, le palmitate de 2-octyl décyle, le myristate de 2-octyl dodécyle, le succinate de 2-diéthylhexyle, le malate de diisostéaryle, le lactate de 2-octyl dodécyle, le triisostéarate de glycérine, le triisostéarate de glycérine, etc.; des huiles de silicone comme les polyméthylsiloxanes, les polyméthylphénylsiloxanes, des polysiloxanes modifiés par des acides gras, des polysiloxanes modifiés par des alcools gras, des polysiloxanes modifiés par des polyoxyalkylènes, des silicones fluorées, etc.; des huiles perfluorées et/ou organofluorées ;des acides gras supérieurs tels que l'acide myristique, l'acide palmitique, l'acide stéarique, l'acide béhénique, l'acide oléique, l'acide linoléique, l'acide linolénique, l'acide isostéarique, des alcools gras supérieurs tels que le cétanol, l'alcool stéarylique, l'alcool oléique etc.;

La phase grasse peut contenir en outre des huiles volatiles, qui s'évaporeront au contact de la peau, mais dont la présence dans la composition cosmétique est utile car elles facilitent l'étalement de la composition lors de l'application sur la peau. De tels agents d'étalement appelés ici huiles volatiles sont généralement des huiles ayant à 25°C une tension de vapeur saturante au moins égale à 0,5 millibar (soit 0,5.10²Pa).
Les huiles volatiles lorsqu'elles sont présentes représentent généralement moins de 10% en poids de la composition finale et moins de 20% en poids du liant gras.
Parmi les huiles volatiles utilisables dans la présente invention on citera par exemple des huiles de silicone telles que l'hexaméthyldisiloxane, le cyclopentadiméthylsiloxane, le cyclotétraméthylsiloxane, des huiles fluorées comme celles commercialisées sous la dénomination Galden (Montefluos) ou des huiles isoparaffiniques telles que celles qui sont commercialisées sous la dénomination ISOPAR (E, G, L ou H).

Les compositions selon l'invention peuvent également comprendre les ingrédients habituellement utilisés en cosmétique parmi lesquels on peut citer les parfums, des adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les matières colorantes, les charges. Les quantités de ces différents adjuvants sont celles classiquement utilisées dans les domaines considérés, et par exemple de 0,01 % à 20 % du poids total de la composition. Ces adjuvants, selon leur nature, peuvent être introduits dans la phase grasse, dans la phase aqueuse et/ou dans les sphérules lipidiques.

Comme solvants utilisables dans l'invention, on peut citer les alcools inférieurs, notamment l'éthanol et l'isopropanol, le propylène glycol. Comme gélifiants lipophiles, on peut citer les argiles modifiées comme les bentones, les sels métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon, les extraits bactériens ou végétaux, notamment d'Aloe Vera.
Comme actifs lipophiles, on peut utiliser le tocophérol (vitamine E) et ses dérivés, les acides gras essentiels, les céramides, les huiles essentielles.
On peut, entre autre, introduire dans les compositions selon l'invention des agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que la vitamine D et ses dérivés, les oestrogènes tels que l'oestradiol, l'acide kojique ou l'hydroquinone ;
- les agents anti-radicaux libres, tels que l'alpha-tocophérol ou ses esters, les superoxyde dismutases, certains chélatants de métaux ou l'acide ascorbique et ses esters.
   Comme charges utilisables dans la présente invention, on peut citer notamment, le talc, la silice, le mica, le nitrure de bore, et comme exemples de charges organiques la poudre de Nylon, la poudre de silicone et la poudre de polyméthylène méthacrylate.

Ces compositions constituent notamment des crèmes teintées transparentes, des fonds de teint, des blush, des rouges à lèvres, des eye-liners, des mascaras, des produits de soin pour les lèvres, des fards à joues, des fards à paupières, de produit pour les ongles.

### EXEMPLES

### Exemple 1 : composition de gel aqueux orange pour le maquillage des lèvres

| Phase A: | |
|---|---|
| Carbomer (nom CTFA) (^{*}) | 1,26% |
| Parahydroxy benzoate de méthyle | 0,04% |
| Eau | 33,7% |
| | |

| Phase B: | |
|---|---|
| Triéthanolamine | 2,1% |
| Eau | 30% |
| | |

| Phase C: | |
|---|---|
| Imidazolidinyl urée | 0,3% |
| Eau | qsp 100 |
| | |

| Phase D | |
|---|---|
| colorant polymérique jaune (^{**}) | 10% |
| colorant polymérique rouge (^{***}) | 10% |

| | |
|---|---|
| (*) homopolymère d'acide acrylique réticulé | |
| (**) polyester colorant comprenant 10% de monomère colorant jaune préparé conformément à l'enseignement du brevet US-4,804,719, en solution aqueuse à 32% de matière active. | |
| (***)polyester colorant comprenant 10% de monomère colorant rouge préparé conformément à l'exemple 1 du brevet US-4,804,719, en solution aqueuse à 29% de matière active. | |

### Mode opératoire:

On chauffe A à 100°C sous agitation lente puis on laisse reposer à température ambiante. On introduit B puis C et finalement D dans A, sous agitation lente à température ambiante.

### Exemple 2 :émulsion huile dans eau comprenant un gel aqueux

| Phase A: | |
|---|---|
| Acide stéarique | 2% |
| Stéarate de glycéryle | 3% |
| Isostéarate de glycéryle | 2% |
| Huile minérale | 8% |
| Conservateur | 0,2% |
| Diméthicone | 4% |
| | |

| Phase B: | |
|---|---|
| Triéthanolamine | 1% |
| | |

| Phase C: | |
|---|---|
| Conservateur | 0,2% |
| Silicate de Magnésium et d'Aluminium | |
| gel à 5% de matière active | 20% |
| colorant polymérique rouge (^{*}) | 10% |
| Gomme de cellulose | 3,5% |
| Lauroyl sarcosinate de sodium | 3,5% |
| Glycérine | 2% |
| Eau | qsp 100 |

| Phase D | |
|---|---|
| Conservateur | 0,3% |
| Eau | 2% |

| | |
|---|---|
| (*) polyester colorant comprenant 10% de monomère colorant rouge préparé conformément à l'exemple 1 du brevet US-4,804,719, en dispersion aqueuse à 29% de matière active. | |

### Mode opératoire:

On prépare les phases A et C séparément, on les chauffe à 80°C puis on les homogénéise au Moritz. On introduit B dans A, puis on verse ce mlange sur C sous agitation.On poursuit l'agitation jusqu'à complet refroidissement.

Les formules des exemples 1 et 2 sont parfaitement stables.

Les compositions des exemples 1 et 2 donnent respectivement un rouge à lèvres et un blush de couleur vive et intense. A l'application, on ne constate aucun problème d'apparition de stries, de tâchage de la peau ou des lentilles oculaires. La coloration est stable à la lumière. Le film de maquillage est brillant. On obtient un maquillage à la fois couvrant, saturé et brillant.

Les deux colorants polymériques de l'exemple 1 sont parfaitement compatibles, le résultat de leur mélange en termes de coloration est parfaitement prévisible.

## Revendications

1. Composition de maquillage des yeux, de la peau, des ongles, des lèvres sous la forme d'un gel aqueux, caractérisée en ce qu'elle comprend au moins un colorant polymérique.

2. Composition selon la revendication 1, caractérisée en ce que le colorant polymérique est choisi parmi les polymères polyester, polyamide, polyuréthanne, polyacrylique, poly(méth)-acrylique, polycarbonate, leurs mélanges.

3. Composition selon l'une quelconque des revendications précédentes, caractérisée en ce que le colorant polymérique est un polyester et résulte de la polymérisation de plusieurs monomères dont:
(i) au moins un résidu acide di-carboxylique;
(ii) au moins un résidu diol ; et
(iii) au moins un monomère colorant.

4. Composition selon l'une quelconque des revendications 1 à 2, caractérisée en ce que le colorant polymérique est un polyuréthanne et résulte de la polymérisation de plusieurs monomères dont:
(i) au moins un résidu di-isocyanate;
(ii) au moins un résidu diol ; et
(iii) au moins un monomère colorant.

5. Composition selon l'une des revendications 1 ou 2, caractérisée en ce que le colorant polymérique est un polymère colorant dispersible dans l'eau ayant des groupes de liaison comprenant au moins environ 20 % en moles de carbonyloxy et jusqu'à environ 80 % en moles de carbonylamido, ledit polymère contenant des groupes sulfonate hydrosolubilisants et ayant d'environ 0,01 à environ 40 % en moles, sur la base du total de tous les équivalents d'hydroxy, de carboxy ou d'amino réactifs, de colorant comprenant un ou plusieurs composés organiques thermostables ayant initialement au moins un groupe condensable, que l'on a fait réagir sur ou dans le tronc polymère.

6. Composition selon l'une quelconque des revendications précédentes, caractérisée en ce que le colorant polymérique comprend 5 % à 55% en poids d'au moins un monomère colorant.

7. Composition selon la revendication 6, caractérisée en ce que le colorant polymérique comprend 10% à 40% en poids d'au moins un monomère colorant.

8. Composition selon l'une quelconque des revendications 3 à 7, caractérisée en ce que le monomère colorant est choisi parmi les anthraquinones, les méthines, bis-méthines, les aza-méthines, les arylidènes, les 3H-dibenzo[7,i-j] isoquinolines, les acides 2,5-diarylaminotéréphtaliques et leurs esters, les phtaloylphénothiazines, les phtaloylphénoxazines, les phtaloylacridone, les anthrapyrimidines, les anthrapyrazoles, les phtalocyanines, les quinophtalones, les indophénols, perinones, les nitroarylamines, benzodifurane, les 2 H-1-benzopyran-2-one, les quinophtalones, les perylènes, les quinacridones, les triphénodioxazines, les fluoridines, les 4-amino-1,8-naphtalimides, les thioxanthrones, les benzanthrones, les indanthrones, les indigo, thioindigo, xanthène, acridine, azine, oxazine.

9. Composition selon l'une quelconque des revendications précédentes, caractérisée en ce qu'elle comporte 0,1 à 20% en poids de colorant polymérique.

10. Composition selon l'une quelconque des revendications précédentes, caractérisée en ce qu'elle comporte en outre au moins un composé choisi parmi le dioxyde de titane et le dioxyde de zinc.

11. Composition selon l'une quelconque des revendications précédentes, caractérisée en ce qu'elle comprend au moins un agent gélifiant choisi parmi les organopolysiloxanes; les polymères ou copolymères acryliques et polyméthacryliques, les polyacrylamides ; les dérivés de polysaccharides, tels que les dérivés cellulosiques comme la carboxy-méthylcellulose, l'hydroxyméthylcellulose, l'hydroxypropylcellulose, la cellulose microcristalline ; les polymères de xanthane, de gellane, de rhamsan, les alginates, la maltodextrine, l'amidon et ses dérivés, l'acide hyaluronique et ses sels, la gomme de karoya, la farine de caroube, les dérivés de guar notamment l'hydropropylguar ;les polyéthylèneglycols (PEG) et leurs dérivés, les polyvinylpyrrolidones et leurs dérivés.

12. Composition selon la revendication 11, caractérisée en ce qu'elle comprend de 0,01 à 50% en poids de gélifiant par rapport au poids total de la composition.

13. Composition cosmétique sous la forme d'une émulsion, caractérisée en ce qu'elle comprend au moins une huile et/ou une cire et au moins un gel selon l'invention.

14. Composition selon l'une quelconque des revendications précédentes, caractérisée en ce qu'elle comporte au moins un émulsionnant.

15. Composition selon l'une quelconque des revendications précédentes, caractérisée en ce que l'émulsionnant est choisi parmi : les sels alcalins d'acides gras en C12-C24, les phosphatides de soja, les phospholipides, les lysophospholipides, les émulsionnants à base de silicone, les alcools gras, les esters de glycérol, les esters de sorbitan, les esters de méthylglycoside et les esters de saccharose.

16. Composition selon l'une quelconque des revendications précédentes, caractérisée en ce que sa viscosité est supérieure à 1 Pa.s.

17. Composition selon l'une quelconque des revendications précédentes, caractérisée en ce que sa viscosité est supérieure à 3 Pa.s.

18. Composition selon l'une quelconque des revendications précédentes, caractérisée en ce qu'elle se présente sous la forme d'un mascara, d'une crème teintée transparente, d'un fond de teint, d'un blush, d'un rouge à lèvres, d'un eye-liner, d'un produit de soin pour les lèvres, d'un fard à joues ou à paupières, d'un produit pour les ongles.

19. Utilisation d'un colorant polymérique selon l'une des revendications 1 à 18, dans une composition de maquillage des yeux, de la peau, des ongles, des lèvres sous la forme d'un gel aqueux, pour former un film coloré sur la peau, les cils, les ongles, les lèvres.

20. Utilisation d'un colorant polymérique selon l'une quelconque des revendications 1 à 18, dans une composition de maquillage des yeux, de la peau, des ongles, des lèvres sous la forme d'un gel aqueux, qui ne colore pas les cils, les yeux, les ongles, les lèvres après le démaquillage.

21. Procédé de maquillage des yeux, de la peau, des ongles, des lèvres, caractérisé par le fait que l'on applique sur les cils, la peau, les ongles, les lèvres une composition selon l'une quelconque des revendications 1 à 18.

22. Utilisation d'un colorant polymérique selon l'une des revendications 1 à 9 dans une composition de maquillage sous la forme d'un gel aqueux qui ne tâche pas et/ou ne forme pas de stries sur les lentilles oculaires.

## Patentansprüche

1. Zusammensetzung zum Schminken der Augen, der Haut, der Nägel, der Lippen in Form eines wäßrigen Gels, dadurch gekennzeichnet, daß sie mindestens ein polymeres Färbemittel enthält.

2. Zusammensetzung nach Anspruch 1, dadurch gekennzeichnet, daß das polymere Färbemittel unter Polyester-, Polyamid-, Polyurethan-, Polyacryl-, Poly(meth)acryl-, Polycarbonat-Polymeren und Gemischen dieser Polymere ausgewählt ist.

3. Zusammensetzung nach einem der vorhergehenden Ansprüche, dadurch gekennzeichnet, daß das polymere Färbemittel ein Polyester ist, der bei der Polymerisation mehrerer Monomere entsteht, zu denen gehören:
(i) mindestens ein Dicarbonsäurerest,
(ii) mindestens ein Diolrest und
(iii) mindestens ein färbendes Monomer.

4. Zusammensetzung nach einem der Ansprüche 1 und 2, dadurch gekennzeichnet, daß das polymere Färbemittel ein Polyurethan ist, das bei der Polymerisation mehrerer Monomere entsteht, zu denen gehören:
(i) mindestens ein Diisocyanatrest,
(ii) mindestens ein Diolrest und
(iii) mindestens ein färbendes Monomer.

5. Zusammensetzung nach einem der Ansprüche 1 und 2, dadurch gekennzeichnet, daß das polymere Färbemittel ein in Wasser dispergierbares polymeres Färbemittel ist, das Bindungsgruppen aufweist, die mindestens etwa 20 Mol-% Carbonyloxy und bis zu etwa 80 Mol-% Carbonylamido umfassen, wobei das Polymer wasserlöslich machende Sulfonat-Gruppen enthält und etwa 0,01 bis etwa 40 Mol-%, auf der Basis der Gesamtmenge aller reaktiven Hydroxy-, Carboxy- oder Amino-Äquivalente, Färbemittel aufweist, das ein oder mehrere wärmebeständige organische Verbindungen umfaßt, die anfänglich mindestens eine kondensierbare Gruppe enthalten, die mit dem oder innerhalb des Polymerrückgrats umgesetzt werden.

6. Zusammensetzung nach einem der vorhergehenden Ansprüche, dadurch gekennzeichnet, daß das polymere Färbemittel 5 bis 55 Gew.-% mindestens eines färbenden Monomers enthält.

7. Zusammensetzung nach Anspruch 6, dadurch gekennzeichnet, daß das polymere Färbemittel 10 bis 40 Gew.-% mindestens eines färbenden Monomers enthält.

8. Zusammensetzung nach einem der Ansprüche 3 bis 7, dadurch gekennzeichnet, daß das färbende Monomer unter Anthrachinonen, Methinen, Bismethinen, Azamethinen, Arylidenen, 3H-Dibenzo[7,i-j]isochinolinen, 2,5-Diarylaminoterephthalsäuren und ihren Estern, Phthaloylphenothiazinen, Phthaloylphenoxazinen, Phthaloylacridonen, Anthrapyrimidinen, Anthrapyrazolen, Phthalocyaninen, Chinophtalonen, Indophenolen, Perinonen, Nitroarylaminen, Benzodifuran, 2H-1-Benzopyran-2-onen, Chinophthalonen, Perylenen, Chinacridonen, Triphenodioxazinen, Fluoridinen, 4-Amino-1,8-naphthalimiden, Thioxanthronen, Benzanthronen, Indanthronen, Indigo, Thioindigo, Xanthen, Acridin, Azin, Oxazin ausgewählt ist.

9. Zusammensetzung nach einem der vorhergehenden Ansprüche, dadurch gekennzeichnet, daß sie 0,1 bis 20 Gew.-% polymeres Färbemittel enthält.

10. Zusammensetzung nach einem der vorhergehenden Ansprüche, dadurch gekennzeichnet, daß sie außerdem mindestens eine Verbindung enthält, die unter Titandioxid und Zinkoxid ausgewählt ist.

11. Zusammensetzung nach einem der vorhergehenden Ansprüche, dadurch gekennzeichnet, daß sie mindestens einen Gelbildner enthält, der unter den Polyorganosiloxanen, den Acryl- und Methacrylpolymeren, den Acryl- und Methacrylcopolymeren, den Polyacrylamiden, den Polysaccharid-Derivaten, wie den Cellulose-Derivaten, wie Carboxymethylcellulose, Hydroxymethylcellulose, Hydroxypropylcellulose, mikrokristalline Cellulose, den Xanthan-Polymeren, Gellan-Polymeren, Rhamsan-Polymeren, den Alginaten, Maltodextrin, Stärke und ihren Derivaten, der Hyaluronsäure und ihren Salzen, Karoyagummi, Carobenmehl, den Guarderivaten, insbesondere Hydroxypropylguar, den Polyethylenglykolen (PEG) und ihren Derivaten, den Polyvinylpyrrolidonen und ihren Derivaten ausgewählt ist.

12. Zusammensetzung nach Anspruch 11, dadurch gekennzeichent, daß sie 0,01 bis 50 Gew.% Gelbildner, bezogen auf das Gesamtgewicht der Zusammensetzung, enthält.

13. Kosmetische Zusammensetzung in Form einer Emulsion, dadurch gekennzeichnet, daß sie mindestens ein Öl und/oder ein Wachs und mindestens ein erfindungsgemäßes Gel enthält.

14. Zusammensetzung nach einem der vorhergehenden Ansprüche, dadurch gekennzeichnet, daß sie mindestens einen Emulgator enthält.

15. Zusammensetzung nach einem der vorhergehenden Ansprüche, dadurch gekennzeichnet, daß der Emulgator unter den Alkalisalzen der C₁₂-C₂₄-Fettsäuren, den Sojaphosphatiden, den Phospholipiden, den Lysophospholipiden, den Emulgatoren auf Siliconbasis, den Fettalkoholen, den Glycerinestern, den Sorbitanestern, den Methylglucosid-Estern und den Saccharoseestern ausgewählt ist.

16. Zusammensetzung nach einem der vorhergehenden Ansprüche, dadurch gekennzeichnet, daß ihre Viskosität größer als 1 Pa·s ist.

17. Zusammensetzung nach einem der vorhergehenden Ansprüche, dadurch gekennzeichnet, daß ihre Viskosität größer als 3 Pa·s ist.

18. Zusammensetzung nach einem der vorhergehenden Ansprüche, dadurch gekennzeichnet, daß sie in Form eines Mascara, einer durchsichtigen gefärbten Creme, eines Make-ups, eines Blushs, eines Lippenstifts, eines Eyeliners, eines Pflegeprodukts für die Lippen, eines Rouges, eines Lidschattens, eines Produkts für die Nägel vorliegt.

19. Verwendung eines polymeren Färbemittels nach einem der Ansprüche 1 bis 18 in einer Schminkzusammensetzung für die Augen, die Haut, die Nägel, die Lippen in Form eines wäßrigen Gels zur Erzeugung eines gefärbten Films auf der Haut, den Wimpern, den Nägeln, den Lippen.

20. Verwendung eines polymeren Färbemittels nach einem der Ansprüche 1 bis 18 in einer Schminkzusammensetzung für die Augen, die Haut, die Nägel, die Lippen in Form eines wäßrigen Gels, das die Wimpern, die Augen, die Nägel, die Lippen nach dem Abschminken nicht mehr anfärbt.

21. Verfahren zum Schminken der Augen, der Haut, der Nägel, der Lippen, dadurch gekennzeichnet, daß auf die Wimpern, die Haut, die Nägel, die Lippen eine Zusammensetzung nach einem der Ansprüche 1 bis 18 aufgetragen wird.

22. Verwendung eines polymeren Färbemittels nach einem der Ansprüche 1 bis 9 in einer Schminkzusammensetzung in Form eines wäßrigen Gels, das keine Flecken hinterläßt und/oder keine Streifen auf Kontaktlinsen bildet.

## Claims

1. Composition for making up the eyes, the skin, the nails or the lips, in the form of an aqueous gel, characterized in that it comprises at least one polymeric dye.

2. Composition according to Claim 1, characterized in that the polymeric dye is chosen from polyester, polyamide, polyurethane, polyacrylic, poly(meth)acrylic and polycarbonate polymers, and mixtures thereof.

3. Composition according to either of the preceding claims, characterized in that the polymeric dye is a polyester and results from the polymerization of several monomers including:
(i) at least one dicarboxylic acid residue;
(ii) at least one diol residue; and
(iii) at least one dyeing monomer.

4. Composition according to either of Claims 1 and 2, characterized in that the polymeric dye is a polyurethane and results from the polymerization of several monomers including:
(i) at least one diisocyanate residue;
(ii) at least one diol residue; and
(iii) at least one dyeing monomer.

5. Composition according to either of Claims 1 and 2, characterized in that the polymeric dye is a water-dispersible dyeing polymer containing bonding groups comprising at least about 20 mol% of carbonyloxy and up to about 80 mol% of carbonylamido, the said polymer containing water-solubilizing sulphonate groups and containing from about 0.01 to about 40 mol%, on the basis of the total of all of the equivalents of hydroxyl, carboxyl or amino reagents, of dye comprising one or more heat-stable organic compounds initially containing at least one condensable group, which has been reacted with or in the polymer trunk.

6. Composition according to any one of the preceding claims, characterized in that the polymeric dye comprises 5% to 55% by weight of at least one dyeing monomer.

7. Composition according to Claim 6, characterized in that the polymeric dye comprises 10% to 40% by weight of at least one dyeing monomer.

8. Composition according to any one of Claims 3 to 7, characterized in that the dyeing monomer is chosen from anthraquinones, methines, bis-methines, aza-methines, arylidenes, 3H-dibenzo[7,i-j] isoquinolines, 2,5-diarylaminoterephthalic acids and esters thereof, phthaloylphenothiazines, phthaloylphenoxazines, phthaloylacridones, anthrapyrimidines, anthrapyrazoles, phthalocyanins, quinophthalones, indophenols, perinones, nitroarylamines, benzodifurans, 2H-1-benzopyran-2-ones, quinophthalones, perylenes, quinacridones, triphenodioxazines, fluoridines, 4-amino-1,8-naphthalimides, thioxanthrones, benzanthrones, indanthrones, indigos, thioindigos, xanthenes, acridines, azines and oxazines.

9. Composition according to any one of the preceding claims, characterized in that it comprises 0.1 to 20% by weight of polymeric dye.

10. Composition according to any one of the preceding claims, characterized in that it also comprises at least one compound chosen from titanium dioxide and zinc dioxide.

11. Composition according to any one of the preceding claims, characterized in that it comprises at least one gelling agent chosen from organopolysiloxanes; acrylic and polymethacrylic polymers or copolymers, polyacrylamides; polysaccharide derivatives, such as cellulose derivatives, for instance carboxymethylcellulose, hydroxymethylcellulose, hydroxypropylcellulose, microcrystalline cellulose; xanthan, gellan or rhamsan polymers, alginates, maltodextrin, starch and its derivatives, hyaluronic acid and its salts, karaya gum, carob flour, guar derivatives, in particular hydroxypropylguar; polyethylene glycols (PEG) and derivatives thereof, and polyvinylpyrrolidones and derivatives thereof.

12. Composition according to Claim 11, characterized in that it comprises from 0.01 to 50% by weight of gelling agent relative to the total weight of the composition.

13. Cosmetic composition in the form of an emulsion, characterized in that it comprises at least one oil and/or one wax and at least one gel according to the invention.

14. Composition according to any one of the preceding claims, characterized in that it comprises at least one emulsifier.

15. Composition according to any one of the preceding claims, characterized in that the emulsifier is chosen from: alkaline salts of C₁₂-C₂₄ fatty acids, soya phosphatides, phospholipids, lysophospholipids, silicone-based emulsifiers, fatty alcohols, glycerol esters, sorbitan esters, methylglycoside esters and sucrose esters.

16. Composition according to any one of the preceding claims, characterized in that its viscosity is greater than 1 Pa.s.

17. Composition according to any one of the preceding claims, characterized in that its viscosity is greater than 3 Pa.s.

18. Composition according to any one of the preceding claims, characterized in that it is in the form of a mascara, a transparent tinted cream, a foundation, a blusher, a lipstick, an eyeliner, a lipcare product, a face powder, an eyeshadow or a nailcare product.

19. Use of a polymeric dye according to one of Claims 1 to 18, in a composition for making up the eyes, the skin, the nails or the lips, in the form of an aqueous gel, to form a coloured film on the skin, the eyelashes, the nails or the lips.

20. Use of a polymeric dye according to any one of Claims 1 to 18, in a composition for making up the eyes, the skin, the nails or the lips, in the form of an aqueous gel, which does not colour the eyelashes, the eyes, the nails or the lips after removal of the make-up.

21. Process for making up the eyes, the skin, the nails or the lips, characterized in that a composition according to any one of Claims 1 to 18 is applied to the eyelashes, the skin, the nails or the lips.

22. Use of a polymeric dye according to one of Claims 1 to 9, in a make-up composition in the form of an aqueous gel which does not mark and/or does not form lines on contact lenses.
